# EUROPEAN PATENT SPECIFICATION

(11) **EP 2 589 351 B1**
(45) Date of publication and mention of the grant of the patent: **24.11.2021**
(21) Application number: 12190897.4
(22) Date of filing: 31.10.2012
(51) Int. Cl.: A61B 17/70

(54) **Facet fixation systems**
Facettenbefestigungssysteme
Systèmes de fixation à facettes

(30) Priority: 01.11.2011 US 201161554218 P; 06.02.2012 US 201213367308; 16.07.2012 US 201261672093 P
(43) Date of publication of application: 08.05.2013
(73) Proprietor: Synergy Disc Replacement Inc., Cambridge ON N1R 8J8 (CA)
(72) Inventor: Duggal, Neil, London, Ontario N6G 2S6 (CA); Hushka, Dylan M, Gilbert, AZ Arizona 85296 (US); Butters, Joshua A, Chandler, AZ Arizona 85224 (US); Slater, Nicholas, Chandler, AZ Arizona 85248 (US)
(74) Representative: HGF

(56) References cited:
- US-A1- 2006 217 713
- US-A1- 2006 293 663
- US-A1- 2008 255 622
- US-A1- 2009 192 551
- US-A1- 2010 069 965
- US-A1- 2011 190 821

## Description

### BACKGROUND OF THE DISCLOSURE

The present disclosure relates to bone and joint fixation and instrumentation and methods for preparation and implantation of these devices. Joint fixation may be necessary in cases of pain and inflammation due to cartilage degeneration, nerve impingement, spinal misalignment, and motion instability. The primary examples described herein illustrate how this concept is applied to the facet joint, but this concept applies equally to other joints where similar causes of pain and inflammation are indicated.
US 2010/0069965 A1 discloses a device that limits extension between a top-most and a bottom-most vertebra within a spinal segment of three or more vertebrae by limiting the distance between the inferior articulating process of the upper-most vertebra and the superior articulating process of the lower-most vertebra. This can be accomplished by anchoring the device onto the intermediate vertebral bone wherein a protrusion of device abuts the inferior articulating process of the upper-most vertebra. The device contains a bore hole adapted to accept a bone screw or other fastener. Spikes are adapted to penetrate the underlying bone surface and prevent device rotation.
US 2008/0255622 A1 discloses a spinal implant including a stabilization member coupled to an elongate member. The implant can be configured for placement within a facet joint in an intra-facet or trans-facet configuration. Also, the implant can include a fusion-promoting bioactive material thereby providing a single device capable of spinal stabilization and/or fusion.

US 2006/0293663 A1 discloses a system for dynamic stabilization of the spine. The system includes a bone engaging fastener configured to be anchored in a vertebra and a deflectable element mounted on the fastener. The deflectable element or bumper is configured and arranged to contact a portion of an adjacent vertebra, such as the facet, during extension of the spine, while offering no resistance during flexion. A cable may be fastened to the bone engaging fastener and arranged to contact a portion of the adjacent vertebra, such as the spinous process, during flexion of the spine.

Facet fixation devices are typically implanted as a minimally invasive means of posteriorly stabilizing the spine. Biomechanical studies have shown that facet screw systems perform similarly or better than pedicle screw systems. Many commercially available facet screws require a complex and timely oblique fluoroscopic technique to achieve the proper placement trajectory. The facet fixation system disclosed herein employs a simple pedicle targeting trajectory requiring only M/L and A/P fluoroscopy. The cap geometry reduces placement sensitivity and allows the surgeon to capture and compress an increased bone surface, while optionally lateralizing the implant to better accommodate interspinous process access.

### SUMMARY OF THE INVENTION

The invention provides a device for facet joint fixation comprises a fastener and a cap, the cap including: a base portion having a circular perimeter; a raised portion protruding from the base portion, the raised portion having a non-circular perimeter, the raised portion including a first lobe and a second lobe, the first lobe wider than the second lobe; and an aperture extending through the base portion and the raised portion, the aperture extension of the spine, while offering no resistance during flexion. A cable may be fastened to the bone engaging fastener and arranged to contact a portion of the adjacent vertebra, such as the spinous process, during flexion of the spine.

Facet fixation devices are typically implanted as a minimally invasive means of posteriorly stabilizing the spine. Biomechanical studies have shown that facet screw systems perform similarly or better than pedicle screw systems. Many commercially available facet screws require a complex and timely oblique fluoroscopic technique to achieve the proper placement trajectory. The facet fixation system disclosed herein employs a simple pedicle targeting trajectory requiring only M/L and A/P fluoroscopy. The cap geometry reduces placement sensitivity and allows the surgeon to capture and compress an increased bone surface, while optionally lateralizing the implant to better accommodate interspinous process access.

### SUMMARY OF THE INVENTION

The present invention relates to a device for facet joint fixation as claimed hereafter. Preferred embodiments are set forth in the dependent claims. Associated methods are also described herein to aid understanding of the invention, but these do not form part of the claimed invention.

The invention provides a device for facet joint fixation comprising a fastener and a cap, the cap including: a base portion having a circular perimeter; a raised portion protruding from the base portion, the raised portion having a non-circular perimeter, the raised portion including a first lobe and a second lobe, the first lobe wider than the second lobe, the widths of the first lobe and second lobe measured in a plane perpendicular to a cap axis; and an aperture extending through the base portion and the raised portion, the aperture shaped to receive the fastener to allow the fastener to fasten the cap to a facet joint to stabilize the facet joint. The cap axis is centrally located relative to the aperture. The base portion includes a plurality of teeth projecting from an attachment surface opposite the raised portion. The raised portion of the cap including one or more connection features shaped to receive a guiding instrument, wherein the one or more connection features are recesses or slots in communication with the aperture.

In an embodiment the aperture is offset from the center of the base portion and / or the aperture extends through the first lobe.

In an embodiment the fastener is a screw, optionally wherein the screw is polyaxially adjustable relative to the cap.

In an embodiment, each of the plurality of teeth has at least one beveled surface. In a further embodiment, the plurality of teeth are arranged on the attachment surface so that the beveled surfaces are oriented inwardly, and wherein at least two opposing beveled surfaces face one another to promote compression across the facet joint when the cap is fastened to the facet joint. In another embodiment the at least two opposing beveled surfaces are on opposing sides of the aperture.

In an embodiment the aperture is circumscribed by an aperture wall, wherein the aperture wall is spherically concave. In a further embodiment the aperture wall has a first end intersecting the raised portion and a second end intersecting the attachment surface, wherein the aperture wall widens at at least one of the first end and the second end.

In an embodiment the connection feature comprises a pair of recesses, and wherein the recesses are on opposite sides of the aperture.

In an embodiment the perimeter of the raised portion is eccentric.

In an embodiment the raised portion is ovoid shaped.

In an embodiment the cap further includes a flared section at the juncture of the base portion and the raised portion, the flared section providing a curved transition between the base portion and the raised portion. For example, in an embodiment the perimeter of the raised portion is eccentric and/or the raised portion is ovoid shaped and/or the cap includes a flared section at the juncture of the base portion and the raised portion, the flared section providing a curved transition between the base portion and the raised portion.

In an embodiment the cap further includes a fenestration, the fenestration providing a pocket for graft material.

In an embodiment the base portion includes an attachment surface opposite the raised portion and the attachment surface is planar, and wherein the raised portion is smoothly rounded to provide a low and minimally obtrusive device profile.

### BRIEF DESCRIPTION OF THE DRAWINGS

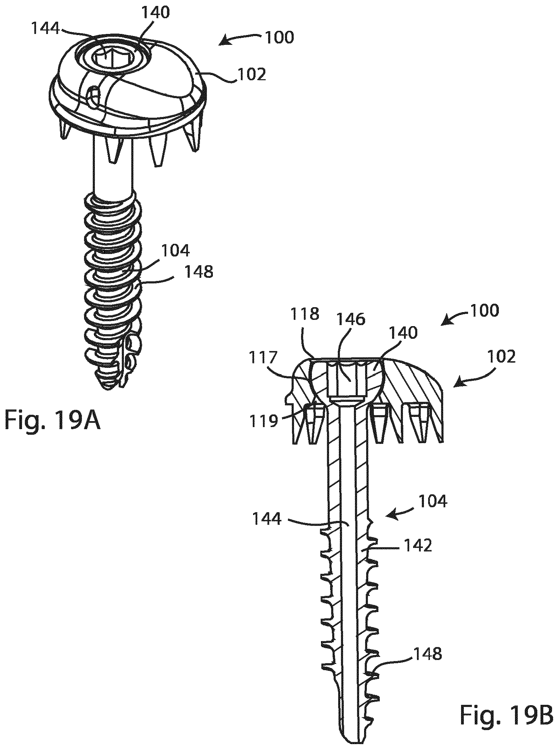

Various embodiments of the present disclosure will now be discussed with reference to the appended drawings. It will be appreciated that these drawings depict only typical examples of the present disclosure and are therefore not to be considered limiting of its scope. FIGS. 19A-B, 20A-B, 21A-B, 24A-C, 25A-D, 27 and 28 illustrate embodiments of the invention. The remaining figures illustrate examples that are useful for understanding the invention.
FIG. 1A is an isometric view of a cap in accordance with one example of the present disclosure;
FIG. 1B is a bottom isometric view of the cap in FIG. 1A;
FIG. 1C is a bottom view of the cap in FIG. 1A;
FIG. 1D is a top view of the cap in FIG. 1A;
FIG. 1E is a back view of the cap in FIG. 1A;
FIG. 1F is a front view of the cap in FIG. 1A;
FIG. 1G is a left side view of the cap in FIG. 1A;
FIG. 1H is a right side view of the cap in FIG. 1A;
FIG. 2A is an isometric view of a cap in accordance with another example of the present disclosure;
FIG. 2B is an isometric view of the cap in FIG. 2A with longer teeth;
FIG. 2C is an isometric view of a cap in accordance with another example of the present disclosure;
FIG. 2D is an isometric view of the cap of FIG. 2C with longer teeth;
FIG. 3 is an isometric view of a cap in accordance with another example of the present disclosure;
FIG. 4 is an isometric view of a cap in accordance with another example the present disclosure;
FIG. 5A shows an implant comprising the cap of FIG. 1A and a fastener before they are assembled together;
FIG. 5B shows the cap of FIG. 1A and the fastener of FIG. 5A after they are assembled together;
FIG. 6 shows a cap and fastener assembly according to another example of the present disclosure;
FIG. 7A shows a portion of a spine with an implant fastened to a facet joint according to the present disclosure;
FIG. 7B shows a back isometric view of the spine and implant of FIG. 7A;
FIG. 8A shows an isometric view of a portion of a spine with an implant fastened to a facet joint in a first orientation;
FIG. 8B shows an isometric view of a portion of a spine with an implant fastened to a facet joint in a second orientation;
FIG. 8C shows an isometric view of a portion of a spine with an implant fastened to a facet joint in a third orientation;
FIG. 8D shows an isometric view of a portion of a spine with an implant fastened to a facet joint in a fourth orientation;
FIG. 8E shows an isometric view of a portion of a spine with an implant fastened to a facet joint in a fifth orientation;
FIG. 9 shows a portion of a cervical spine with an implant fastened to a facet joint according to the present disclosure;
FIG. 10 shows an isometric view of a dilator in accordance with one example of the present disclosure;
FIG. 11 shows an isometric view of a cannula in accordance with one example of the present disclosure;
FIG. 12 shows the dilator of FIG. 10 inserted into the cannula of FIG. 11;
FIG. 13 shows an isometric view of a manual reamer in accordance with one example of the present disclosure;
FIG. 14 shows an isometric view of a powered reamer in accordance with another example of the present disclosure;
FIG. 15A shows an isometric view of a guide in accordance with one example the present disclosure;
FIG. 15B shows a front view of the guide of FIG. 15A;
FIG. 16A shows an isometric view of a guide in accordance with another example the present disclosure;
FIG. 16B shows an isometric view of the guide of FIG. 16A with a cap inserted into the guide;
FIG. 16C shows an isometric view of the guide of FIG. 16A with a cap and fastener assembly inserted into the guide;
FIG. 17A shows an isometric view of a guide in accordance with another example the present disclosure;
FIG. 17B shows an isometric view of the guide of FIG. 17A with a cap inserted into the guide;
FIG. 17 C shows an isometric view of the guide of FIG. 17A with a cap and fastener assembly inserted into the guide;
FIG. 18 shows an isometric view of a fastener driver in accordance with one example of the present disclosure;
FIG. 19A is an isometric view of a facet fixation device having a facet fixation cap with a circular footprint and a screw; FIG. 19B is a side cross-sectional view of the facet fixation device of FIG. 19A;
FIG. 20A is an isometric view of an embodiment of the facet fixation cap of FIG. 19A; FIG. 20B is a side view of the cap of FIG. 20A;
FIG. 21A is a superior view of the cap of FIG. 20A; FIG. 21B is an inferior view of the cap of FIG. 20A;
FIG. 22A is an isometric view of an example of a facet fixation cap having a spiral footprint; FIG. 22B is another isometric view of the cap of FIG. 22A; FIG. 22C is a side view of the cap of FIG. 22A;
FIG. 23A is a superior view of the cap of FIG. 22A; FIG. 23B is an inferior view of the cap of FIG. 22A;
FIG. 24A is an isometric view of another embodiment of a facet fixation cap having a circular footprint; FIG. 24B is superior view of the cap of FIG. 24A; FIG. 24C is a side view of the cap of FIG. 5A;
FIG. 25A is an isometric view of another embodiment of a facet fixation cap having a circular footprint; FIG. 25B is a superior view of the cap of FIG. 25A; FIG. 25C is a side view of the cap of FIG. 25A; FIG. 25D is an inferior view of an alternative embodiment of the cap of FIG. 25A;
FIG. 26 is an isometric view of a facet fixation device including the cap of FIG. 22A and a screw;
FIG. 27 is an isometric view of a facet fixation device including the cap of FIG. 24A and a screw;
FIG. 28 is an isometric view of a facet fixation device including the cap of FIG. 25A and a screw.

### DETAILED DESCRIPTION

Those of skill in the art will recognize that the description is merely illustrative of the principles of the disclosure, which may be applied in various ways to provide many different alternative embodiments and may be applicable outside the fields of surgery or medical devices. While the present disclosure is made in the context of facet joints in the lumbar spinal region for the purposes of illustrating the concepts of the design, it is contemplated that the present design and/or variations thereof may be suited to other uses, such as cervical facet joints, thoracic facet joints, other joints in the human body, or to stabilize bone fractures, etc. Moreover, the implants, instrumentation and methods (not claimed) set forth

herein may be used in open, percutaneous, and/or minimally invasive procedures and may be placed via intra-facet, trans-facet, trans-laminar, or trans-pedicle means.

Standard medical planes of reference and descriptive terminology are employed in this specification. A sagittal plane divides a body into right and left portions. A mid-sagittal plane divides the body into equal right and left halves. A coronal plane divides a body into anterior and posterior portions. A transverse plane divides a body into superior and inferior portions. Anterior means toward the front of the body. Posterior means toward the back of the body. Superior means toward the head. Inferior means toward the feet. Medial means toward the midline of the body. Lateral means away from the midline of the body. Axial means toward a central axis of the body. Abaxial means away from a central axis of the body. Ipsilateral means on the same side of the body. Contralateral means on the opposite side of the body. These descriptive terms may be applied to an animate or inanimate body.

While certain embodiments and other examples are shown and described in detail below by way of illustration only, it will be clear to the person skilled in the art upon reading and understanding this disclosure that changes, modifications, and variations may be made and remain within the scope of the technology described herein. Furthermore, while various features are grouped together in the embodiments and other examples for the purpose of streamlining the disclosure, it is appreciated that features from different embodiments and examples may be combined to form additional embodiments and examples which are all contemplated within the scope of the disclosed technology.

Not every feature of each embodiment or example is labeled in every figure in which that embodiment or example appears, in order to keep the figures clear. Similar reference numbers (for example, those that are identical except for the first numeral) may be used to indicate similar features in different embodiments and examples.

Any of the devices described herein may be fabricated from metals, alloys, polymers, plastics, ceramics, glasses, composite materials, or combinations thereof, including but not limited to: PEEK (polyether ether ketone), titanium, titanium alloys, commercially pure titanium grade 2, ASTM F67, Nitinol, cobalt chrome, stainless steel, UHMWPE (ultrahigh molecular-weight polyethylene) and biodegradable materials, among others. Different materials may be used within a single part. The implants disclosed herein may also encompass a variety of surface treatments or additives to encourage bony attachment, including but not limited to: porous coatings, hydroxyapatite, TCP (tricalcium phosphate), anti-microbial additives, analgesics, anti-inflammatories, BMPs (bone morphogenic proteins), PMA (phorbol myristate acetate) material, bone growth promoting material, PLLA (poly-L-lactide), PGA (polyglycolide), TCP (tricalcium phosphate), demineralized bone, cancellous bone chips, etc. Any implant disclosed herein may include a radiographic marker for imaging purposes. Any implant disclosed herein may be colored, coded or otherwise marked to make it easier for the surgeon to identify the type and size of the implant.

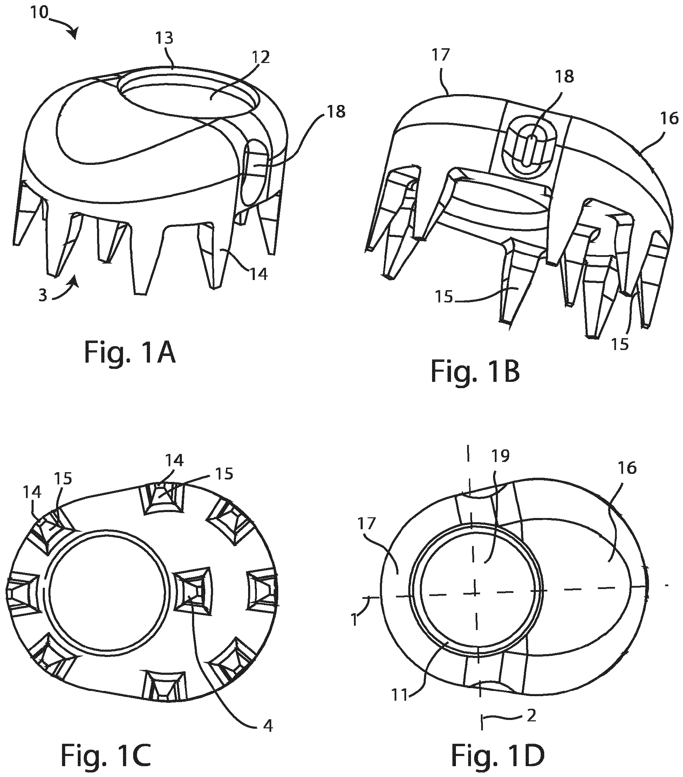

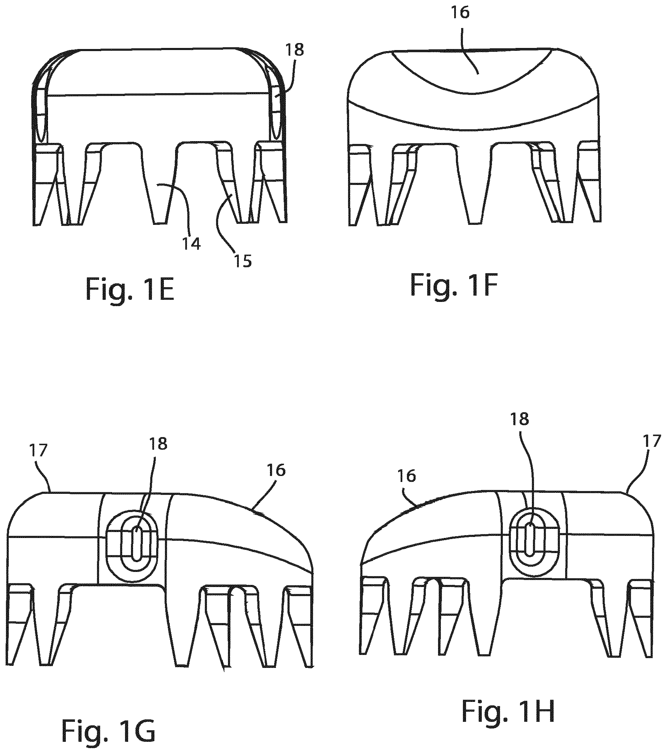

FIGS. 1A-1H illustrate one example of a cap member useful for fixing a bone fracture or joint to provide stabilization. The cap members disclosed herein may be referred to as caps or washers. The cap 10 can have a first portion 16 and a second portion 17. Referring to FIG. ID, the first portion 16 can be longer than the second portion 17 along a first axis 1 which intersects the first portion, the second portion, and an aperture 19 formed in the cap 10. In some examples, the first portion 16 may also be longer then the second portion 17 along a second axis 2. This creates an eccentrically shaped cap 10 with the first portion 16 being asymmetrically shaped in comparison to the second portion 17. The shape of the cap 10 can also be referred to as "oblong" in some examples, with the first portion 16 forming a lobe that is larger than the second portion 17. The eccentric shape of the cap 10 allows a surgeon more freedom to orient the larger lobe portion across the joint to facilitate joint fixation and increases the load bearing area of the implant 10. In some examples, the cap 10 can also curve downward to create a lower profile implant. For example, FIGS. 1G and 1H show left and right side views of the cap 10 with the first portion 16 of the cap curving downward.

The cap 10 can include one or more teeth 14 on a bone engaging side 3 of the cap 10. The plurality of teeth 14 can have beveled surfaces 15 that are arranged to at least partially oppose each other between the first portion 16 of the cap 10 and the second portion 17 of the cap 10. The beveled surfaces 15 can be made to diverge away from each other in the superior to inferior direction and converge toward each other in the inferior to superior direction. In this manner, the beveled surfaces 15 can act to compress the joint bones together as the teeth 14 are driven into the bones. The angle of the beveled services 15 can be adjusted to increase or decrease the compressive forces created by the cap 10. For example, if the angle of the beveled surfaces 15 is increased, the teeth can impart a greater compressive force for a given distance that the teeth 14 are driven into the bones. Thus, the size, length, bevel shape, bevel angle, and distribution of the teeth may vary in any of the examples disclosed herein. For example, the number and spacing of the teeth 14 can be chosen to maximize the fixation properties of the cap 10 in view of the size and condition of the joint bones of the patient. In some examples, the teeth 14 can be distributed on the bone engaging side 3 of the cap 10 along the outer perimeter of the bone engaging side 3 of the cap 10. In other examples the teeth 14 can be distributed away from the outer perimeter of the bone engaging side 3 of the cap 10. For example, FIG. 1C has a tooth 4 which does not lie along the outer perimeter of the bone engaging side 3 of the cap 10, rather tooth 4 is located deeper within the interior of the first portion 16. Having teeth distributed in this manner can increase the bone grabbing performance of the implant by increasing the number of teeth within the interior of the first portion 16.

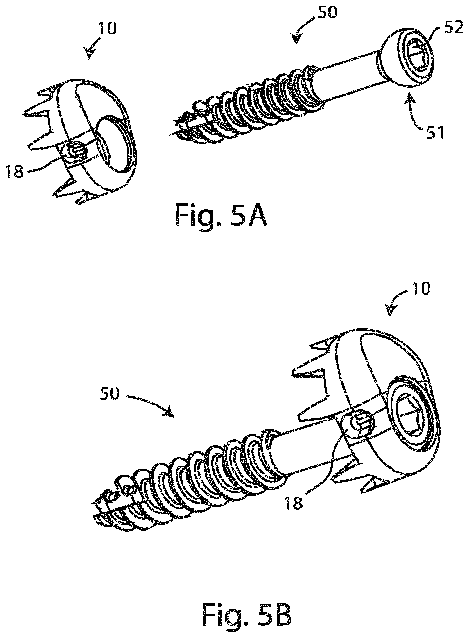

Continuing with FIGS. 1A and ID, the cap 10 can have an aperture 19 formed through the cap 10 and configured to receive a suitable fastener 50, as can be seen in FIGS. 5A-5B. The shaft of the fastener 50 may be partially threaded to promote compression. The fastener 50 can also be self-tapping (or self-threading) and may be cannulated down its center so that it can be placed into the facet joint with a K-wire. The cap 10 can have a chamfered spherical capsule 12 shaped to receive a complimentarily shaped partially spherical fastener head 51, as shown in FIG. 5A. This allows the fastener 50 to rotate within the aperture 19 and concentrically pivot along its longitudinal axis to ensure that the cap 10 can align itself with the joint bones as the cap 10 is forced into the joint bones. The aperture 19 can also be deep enough to allow the fastener head 51 to be recessed within the aperture 19 to provide a smooth, low profile implant. A smooth, low profile implant can help reduce irritation to surrounding soft tissue. The aperture 19 may also be encircled by a lip 13 which projects inward and has a diameter slightly smaller in size than the diameter of the head portion 51 of the suitable fastener 50. This can allow the fastener to be "press fit" into the aperture such that the lip 13 provides an interference that captures the fastener 50 within the aperture 19. The lip 13 can be flush with the surrounding surface of the implant to avoid any abrupt changes in the shape of the implant resulting in smooth surfaces. Thus, the smooth lip 13 that sits flush with the surrounding surface will help reduce irritation to surrounding soft tissues, as compared to other interference fit configurations, such as collet style interference structures which have multiple slits and protruding structures that can cause interference and irritation to surrounding soft tissues and bones.

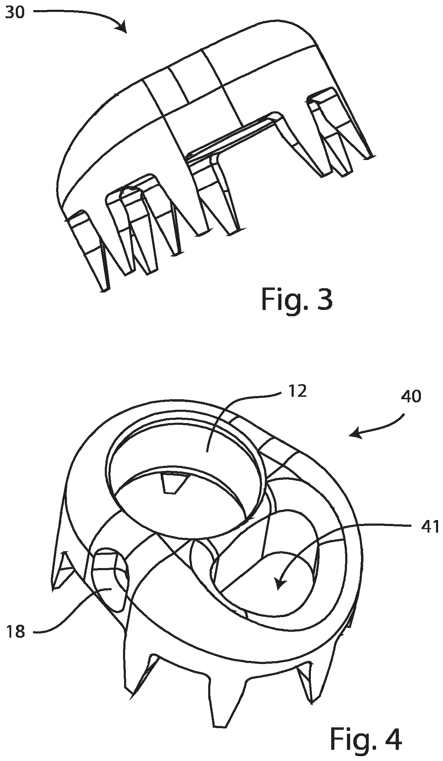

Continuing with FIGS. 1A-1H, the cap 10 can have one or more recessed slots 18 formed in a surface of the cap 10. The slots 18 can interact with a guide tool to hold the cap 10 in a specific orientation during insertion. In other examples, the cap 10 may not include one or more slots 18 formed in a surface of the cap 10. One such example can be seen in FIG. 3.

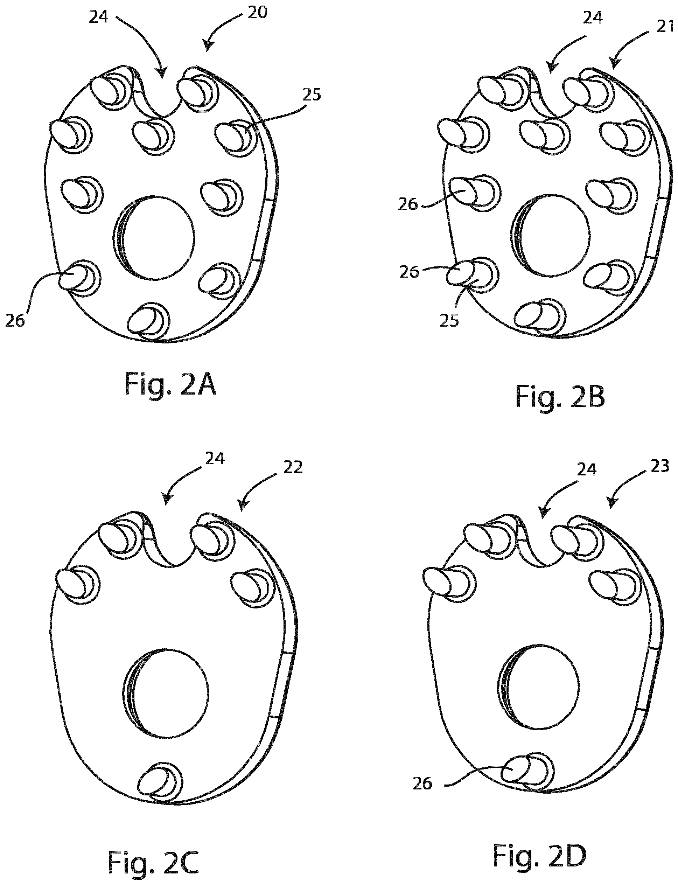

FIGS. 2A-2D illustrate alternative examples of caps 20, 21, 22, and 23 which can be used to fix bones or joints according to other examples of the present disclosure. Each of the caps 20, 21, 22, and 23 may include one or more slots 24 configured to interact with a guide tool to hold the cap at a specific orientation during insertion. However, in other examples, the cap may not include one or more slots 24. It will be appreciated that the location of the one or more slots 24 around the perimeter of the caps may vary, as may the size, diameter and/or number of the one or more slots 24. The one or more slots 24 may cooperate with a suitable guide for properly aligned placement of the cap into the joint, as will be discussed in greater detail below. The caps 20, 21, 22, and 23 can also include a plurality of teeth 25 that can be cylindrical in shape and have varying lengths. The teeth 25 can also include opposing beveled surfaces 26 similar to other examples disclosed herein.

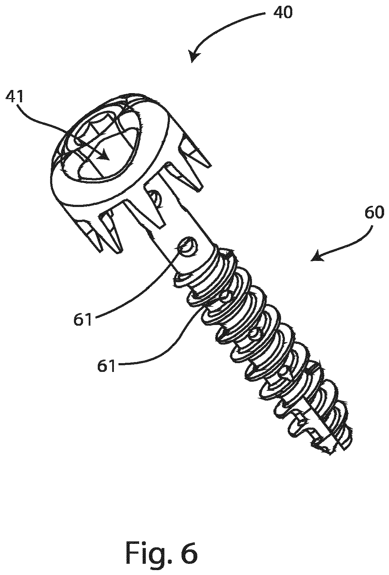

Referring now to FIG. 4, a cap 40 is shown with an aperture or fenestration 41 formed through the cap 40 and configured to promote bone growth, or bone fusion, by providing a graft pocket for material such as bone chips or bone growth promoters. FIG. 6 illustrates the cap 40 in combination with a fastener 60, which is also fenestrated with apertures 61 throughout the fastener 60 which can also be packed with bone chips or bone growth promoters. This combination may further promote bony ingrowth and bone fusion between the fastener 60, the cap 40, and the bones.

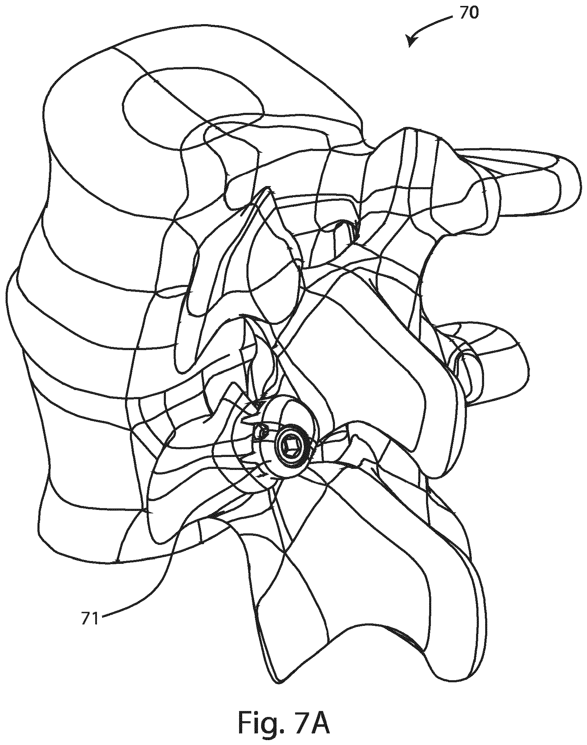

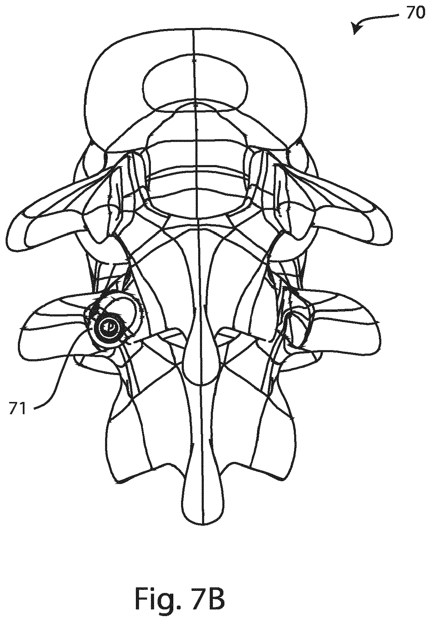

FIGS. 7-9 show various implants affixed to facet joints in portions of the spine. FIG. 7A shows an isometric view of an implant 71 affixed to a facet joint in a lumbar portion of a spine 70. FIG. 7A shows a back isometric view of the implant 71 affixed to the lumbar portion of the spine 70 in FIG. 7.

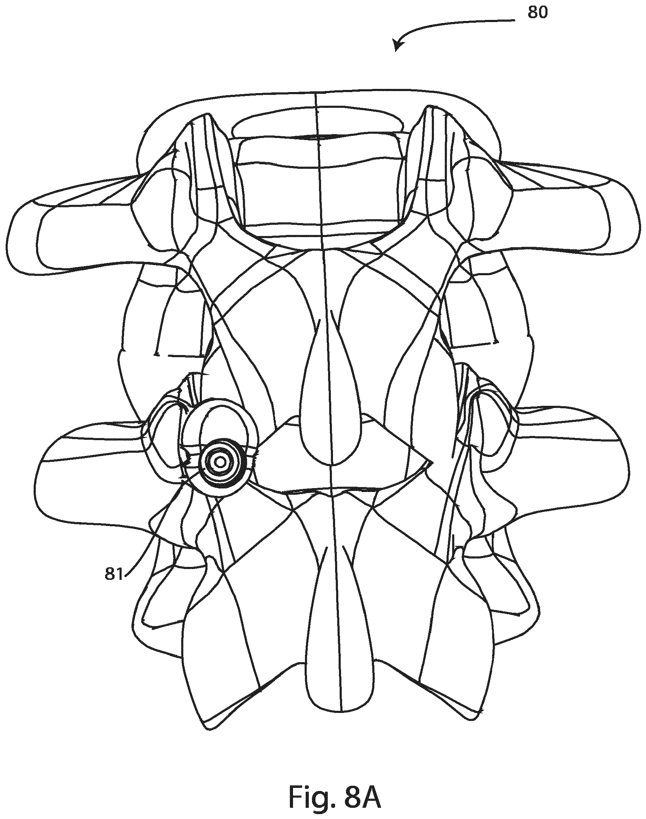

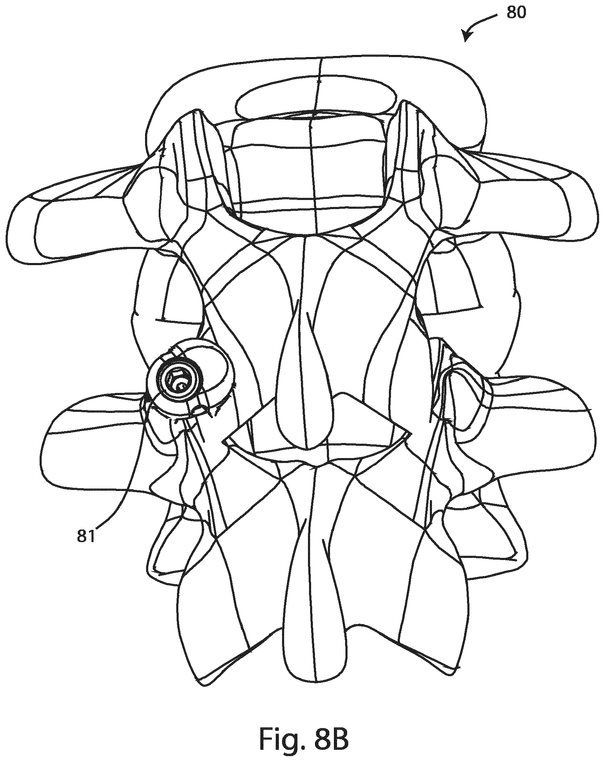

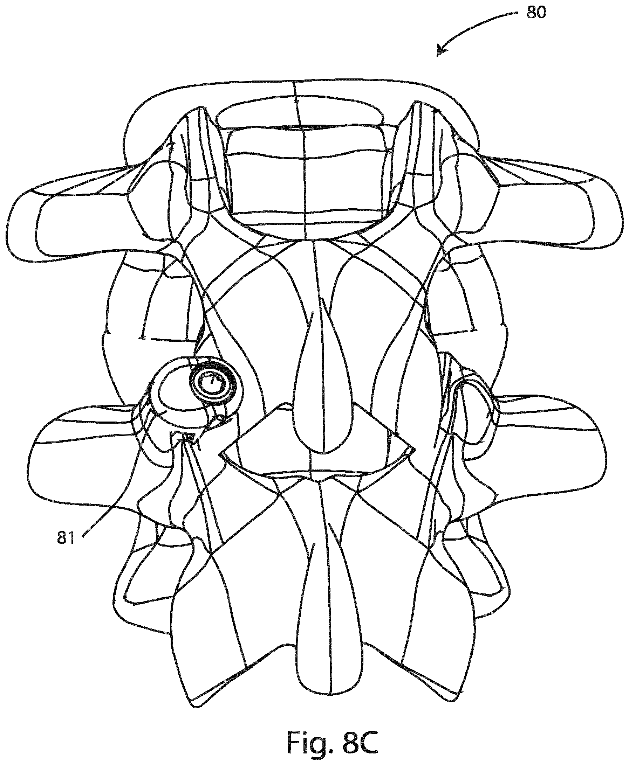

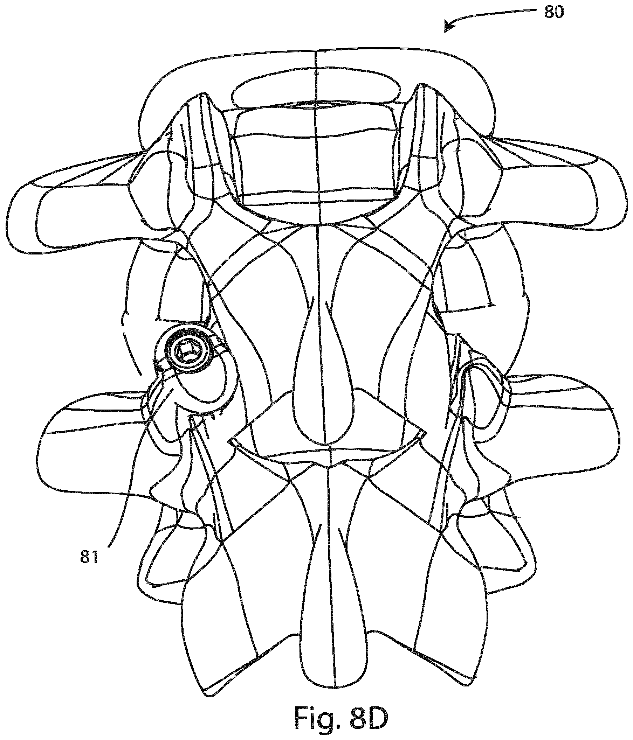

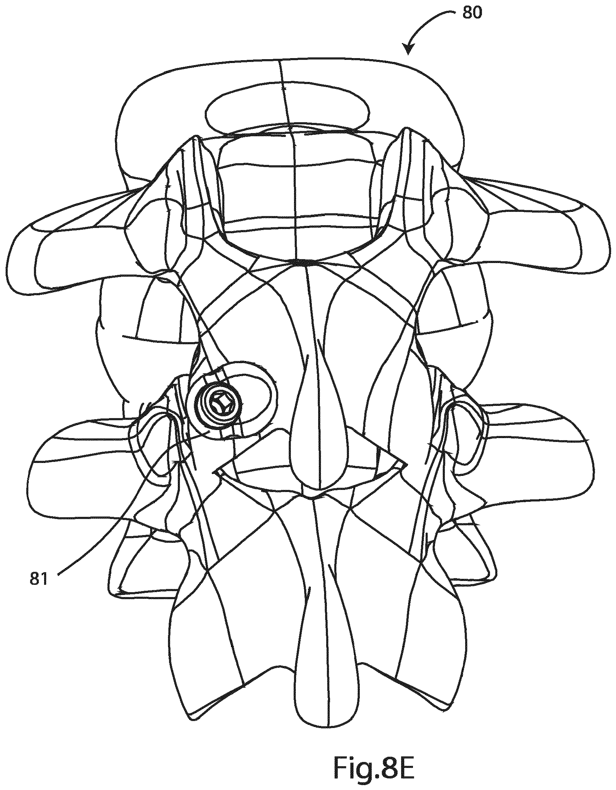

FIGS. 8A-8E show examples of various placement options for an implant 81 in a portion of a spine 80, all of which are easily achievable with the guides and instrumentation disclosed herein. FIG. 8A shows the cap 81 with the fastener piercing the lower or inferior part of the superior articular process and the lobe of the cap oriented superiorly to capture the inferior articular process with the lobe of the cap 81. FIG. 8B shows the cap 81 with the fastener piercing the lateral or middle part of the superior articular process and the lobe of the cap oriented medially to capture the inferior articular process with the lobe of the cap 81. FIG. 8C shows the cap 81 with the fastener piercing the inferior articular process (transfacet) and the lobe of the cap is inverted or oriented laterally to capture the superior articular process with the lobe of the cap 81. FIG. 8D shows the cap 81 with the fastener piercing the upper or superior part of the superior articular process and the lobe of the cap is oriented inferiorly to capture the inferior articular process with the lobe of the cap 81. FIG. 8E shows the cap 81 with the fastener piercing the inferior articular process (transfacet) and the lobe of the cap 81 is oriented medially to capture the inferior articular process with the lobe of the cap 81. It is appreciated that any of the implants disclosed herein can be implanted in any of the orientations disclosed in Figures 8A-8E, including implants with circular footprints or perimeters.

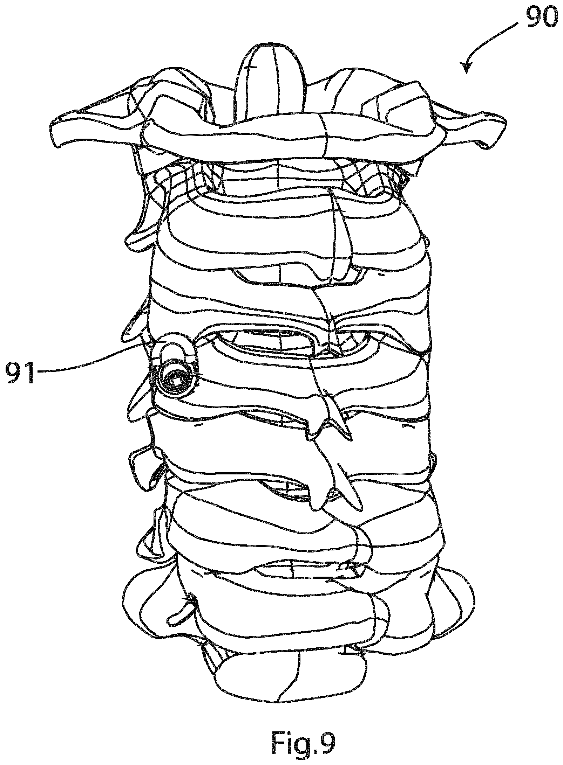

FIG. 9 shows an isometric view of an implant 91 affixed to a facet joint in a cervical portion of the spine 90 demonstrating that the implants disclosed herein can be used in all portions of the spine as well as in other parts of the body.

Methods (not claimed) of inserting the implants disclosed herein will now be given. These methods may be used with any of the implants including caps 10, 20, 21, 22, 23, 30, 40, and implants 71, 81, 91, 100, 200, 300, 400. The methods disclosed herein do not form part of the invention but represent disclosure that is useful for understanding how devices of the invention may be used.

A K-wire can be inserted into the portion of the facet joint where the surgeon desires to affix the fastener to the facet joint. In one example, the K-wire can be inserted into the inferior facet joint and oriented such that the fastener will enter into the pedicle of the inferior facet joint.

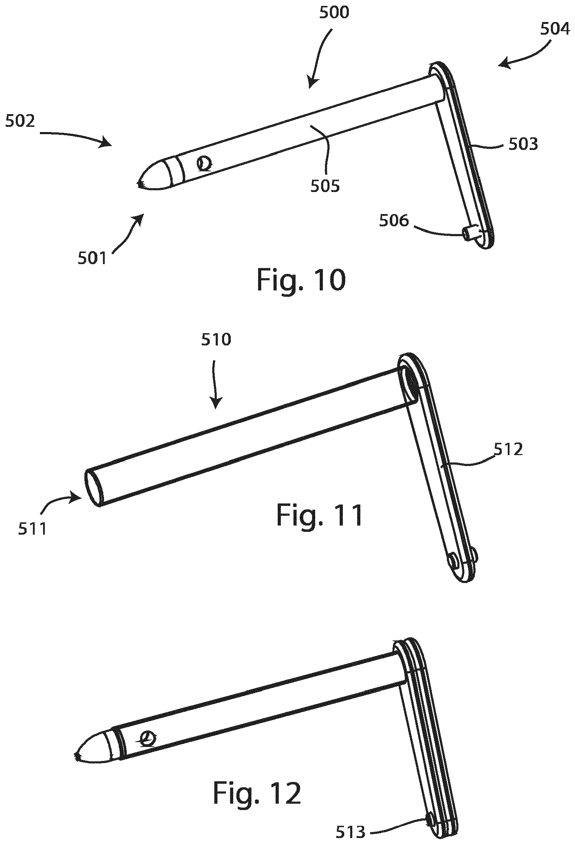

Once the K-wire is in the desired location, a dilator 500 and cannula 510 assembly can be threaded over the K-wire and inserted into the soft tissue of the patient to provide sufficient access to the facet joint. FIG. 10 shows an isometric view of a dilator 500 according to one example of the present disclosure. The dilator 500 can have a pointed tip 501 at its distal end 502 and a handle portion 503 at its proximal end 504. The dilator 500 can also have a shaft 505 having a diameter slightly less than the inside diameter of the hollow shaft 511 of a cannula 510 as seen in FIG. 11. The dilator 500 can be inserted into the cannula 510 as shown in FIG. 12, and the handles 503, 512 of the dilator 500 and the cannula 510 can also align with and engage each other via a boss 506 attached to the handle 503 of the dilator 500 and an aperture 513 formed in the handle 512 of the cannula 510.

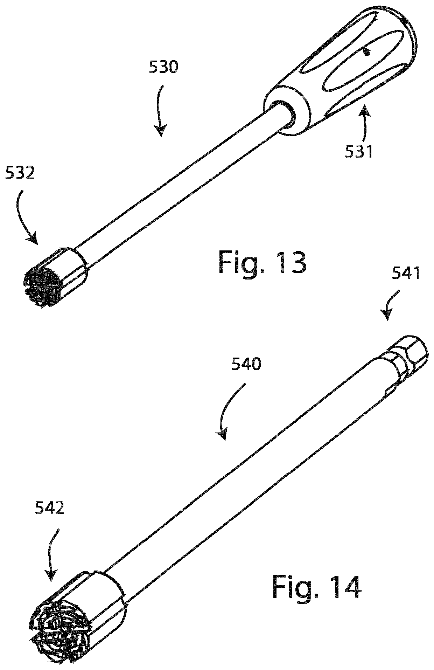

Once the tissue is dilated, the surgeon can remove the dilator 500 from the cannula 510 thus exposing the facet joint through the cannula for the remainder of the surgery. The surgeon may then ream the bone surface of the facet joint with a suitable reamer 530, 540 to prepare the bone surface for receiving the implant. The reamer 530 shown in FIG. 13 is a manual reamer with a handle 531 and a reamer head 532. The reamer 540 shown in FIG. 14 is a powered reamer with a connection 541 configured to receive a suitable power tool and a reamer head 542.

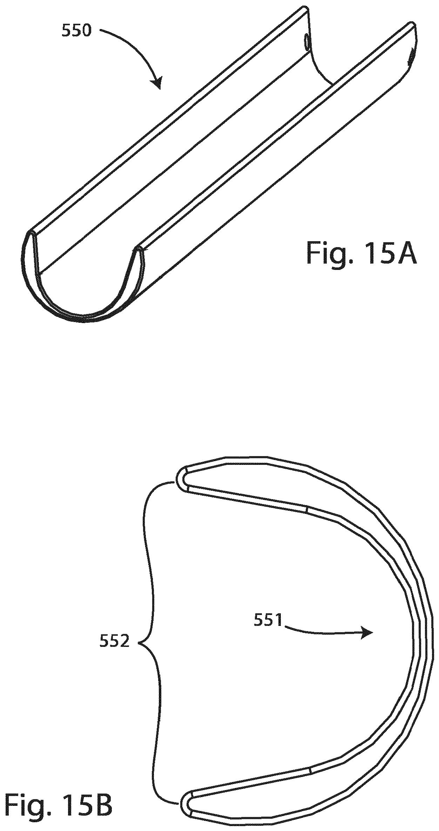

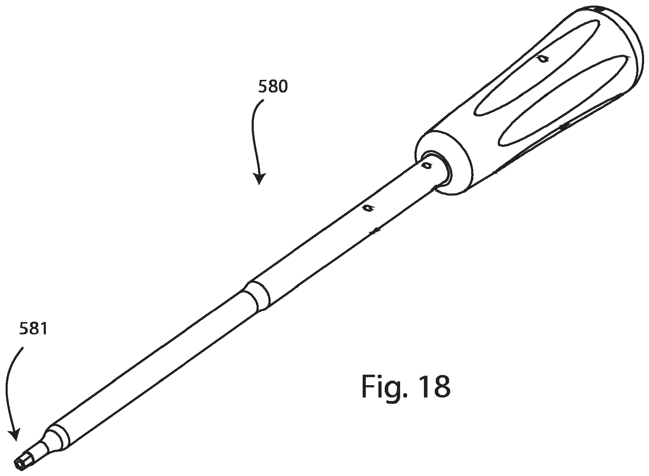

Once the implant site is sufficiently prepared to receive the implant, one or more guides can be used to orient and insert a suitable implant, as can be seen in FIGS. 15A-17C. FIGS. 15A-15B illustrate one example of a guide 550 that may be used with an implant 30 shown in FIG. 3. The implant 30 does not have any slots to engage a portion of the guide 550, as other examples disclosed herein. Rather, the guide 550 is shaped to receive the smaller second portion of the cap 30 in the smaller inner portion 551 of the guide 550 and the larger first portion of the cap 30 in the larger inner portion 552 of the guide 550, as is shown in the front view of the guide in FIG. 15B. The guide 550 is a semi-tubular or semicylindrical member. The outer diameter of the guide 550 may be round to complementarily fit within the cannula 510, but the inner diameter can have a unique cutout profile to accommodate a cap with a variable diameter, such as caps 10, 20, 30, 40. In the example shown, the guide 550 may not be quite a half-pipe as it sweeps close to 245°. Other guide examples may vary in size and shape to accommodate the geometry of other cap examples and embodiments, including caps 10, 20, 21, 22, 23, 40, 102, 202, 302, 402, and 402'. The inner diameters of the cannula 510 and guide 550 match the two different outer diameters of the cap 30; other guides may match the outer diameters of the lobes or perimeters of the other caps. This provides control for proper placement of the cap 30. Thus, the guide 550 is shaped to cooperate with the asymmetrical or eccentric geometry of the cap 30 to guide the cap 30 into place. The guide 550 can be inserted into the cannula 510 and a cap 30 with a suitable fastener attached thereto can be affixed to a suitable driver 580, such as that shown in FIG. 18. The cap 30 can then be inserted into the guide and moved toward the implant site. The shape of the guide 550 in combination with the shape of the implant 30 keeps the cap 30 in the proper orientation as the surgeon slides the cap 30 toward the implant site, and as the fastener is driven into the bone. Caps having a circular base perimeter such as caps 102, 202, 302, 402, and 402' may be implanted with a guide 550 having a circular inner diameter; alternately the guide 550 may not be used and the cap may be implanted directly through the cannula 510.

The driver 580 can have a hexagonal tip 581 configured to interact with a hexagonal aperture 52 as seen in FIG. 5A. The hexagonal aperture 52 can also be chamfered to help the driver 580 stay engaged with the fastener 50.

In one method of implantation, the cap 30 may be placed first, allowing the teeth to capture bone surfaces on both sides of the joint, followed by placement of the screw 30 to provide compression and stability. As the fastener head 51 engages in spherical capsule 12, the teeth 14 are driven into the bone; the beveled surfaces 15 of the teeth promote compression across the facet joint.

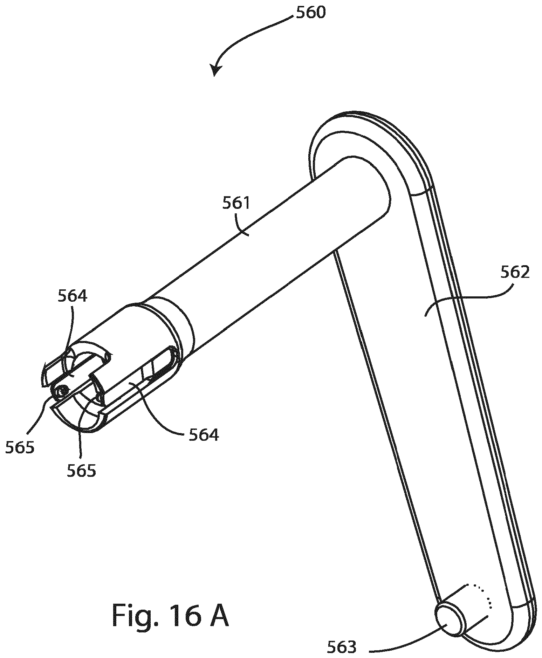

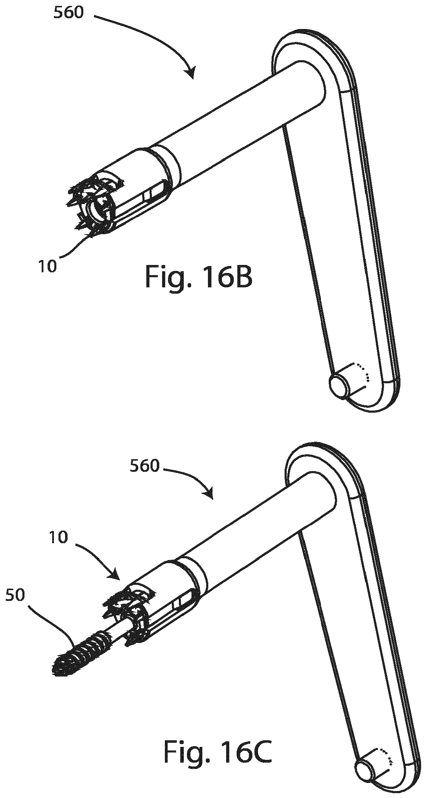

FIGS. 16A-16C show an alternative example of a guide for use with other implants described herein. The guide 560 can have a shaft 561 and a handle 562 with a boss attached to the handle 562. The shaft 561 can be hollow and can include one or more retaining members 564 engaged with the distal end of the hollow shaft 561. Moreover, the retaining members 564 can include boss members 565 sized and shaped to engage suitably shaped recessed slots 18 formed in the cap 10. In other examples, the cap 10 may include other features to cooperate with the one or more retaining members 564, such as recesses, dimples, or grooves. The boss members 565 may be oriented to be offset from each other at the distal end of guide 560 in order to match the offset or eccentric shape of the slots 18 in the cap 10. Guide 560 may also be used to implant devices 100, 200, 300, and 400; boss members 565 can be received in the connection features of caps 102, 202, 302, 402 to guide the caps toward the implantation site and hold the implant while the fastener 104, 204, 304, 404 is engaged in the bone.

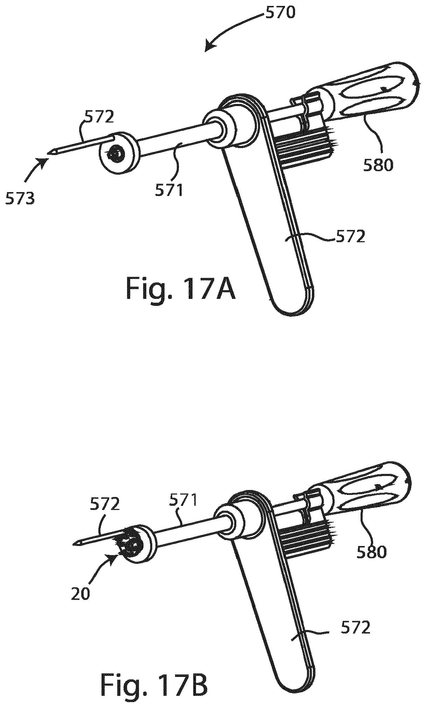

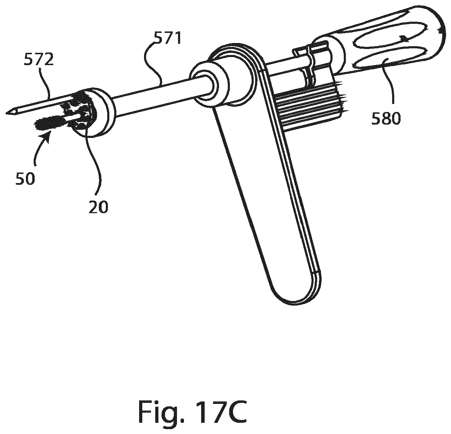

FIG. 17A-17C show yet another example of a guide for use with implants disclosed herein. The guide 570 can have a hollow shaft 571 and a guide pin 572. The guide pin 572 may be offset from a central longitudinal axis of the guide 570. The guide pin 572 cooperates with the slot 26 on the cap 20 to guide the cap 20 along a selected path into proper alignment with the joint. Guide pin 572 may also include a tip 573 which can act as a probe to aid in referencing the joint space. FIG. 17B shows the guide 570 with a cap 20 engaged with the guide 570 via a driver 580 and the guide pin 572. FIG. 17C shows the guide 570 engaged with fastener 50 and cap 20 with the driver 580 pushing the cap 20 and fastener 50 in the distal direction along the guide pin 572. FIG. 18 shows an isometric view of the driver 580 with a hexagonal tip 581. In other examples, the hexagonal tip 581 may be replaced with another shaped drive feature for connection with a suitable fastener. Any of the other cap examples disclosed herein may include slots 26 to allow use of guide 570 during an implantation procedure.

All of the above guides can be used to orient, steer, and insert the implant to the desired location at the implant site where the driver 580 can then be used to apply a torsional rotation force to the fastener 50 to fasten the implant to the joint to stabilize the joint. Once the implant is in the proper location, the surgeon can remove the guide, the driver 580, the cannula 510, and the K-wire and then close the incision site.

Referring to Figures 19A and 19B, an embodiment of a facet fixation device is shown. Device 100 includes a cap 102 and a fastener which in this embodiment is a screw 104. Device 100 and the other facet fixation devices herein may be referred to as an implant. When implanted, screw 104 may extend through cap 102 and through a facet joint, fastening cap 102 to the facet joint and stabilizing the joint. The cap 102 may span the joint, with a portion of the cap affixed to the inferior articular process of a facet joint, and another portion of the cap affixed to the superior articular process of the facet joint. The width of the cap 102 provides increased load bearing area for the screw 104.

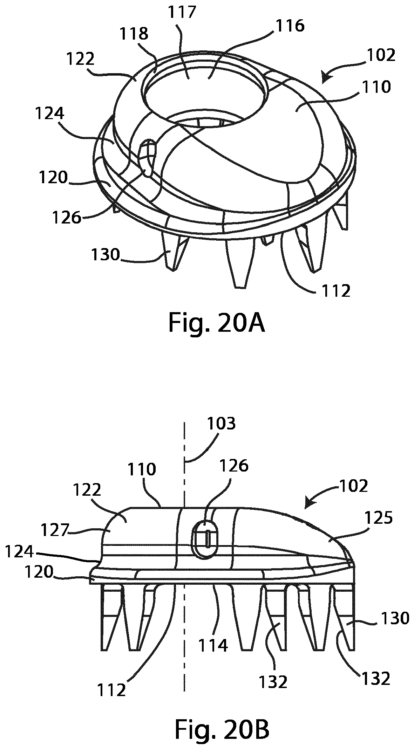

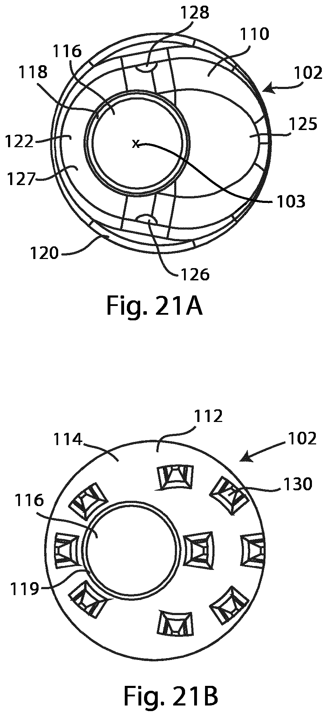

Referring to Figures 20A, 20B, 21A and 21B, cap 102 includes a first side 110 and a second side 112, which may be superior and inferior sides, respectively. The first side 110 may be smoothly rounded to provide a low and minimally obtrusive device profile. The second side 112 includes an attachment surface 114. In the embodiment shown, attachment surface 114 is circular, and planar, although in other embodiments the attachment surface may be non-planar. An aperture 116 extends through the cap 102 between the first side 110 and the second side 112. A cap axis 103 is centrally located relative to the aperture 116. The aperture 116 is also circular, although in other embodiments the aperture 116 may take another shape. The aperture 116 is offset, or eccentrically positioned relative to the geometric center of the cap 102 and the outer circular footprint of the cap. Aperture 116 is circumscribed by an aperture wall 117, which is spherically concave or cup-shaped to receive the head of screw 104. In other embodiments, the aperture wall may be flat or conically concave. Toward the first side 110, aperture wall 117 widens at a first flared end 118 and toward the second side 112, aperture wall 117 widens at a second flared end 119, best seen in Figure 19B. In another embodiment, cap 102 may include a second aperture 41 which is a fenestration to provide a graft pocket for material such as demineralized bone material, graft material, bone chips or bone growth promoters.

The cap 102 further includes a base portion 120 and a raised portion 122. The base portion 120 is shaped as a circular flange with a circular perimeter, and the raised portion 122 projects superiorly from the base portion 120. In the context of this disclosure, a perimeter is a line bounding a cap portion at its largest extent, the line bounding a plane perpendicular to the cap axis. While the base portion 120 is circular with a circular perimeter, the raised portion 122 is not radially symmetrical and has a non-circular, eccentric perimeter, and in the embodiment shown is ovoid, having a wider first end 125 and a relatively narrower second end 127. The first and second ends 125, 127 may be lobes of the raised portion 120; the widths of the lobes 125, 127 are measured in a plane perpendicular to the cap axis 103. The second end or lobe 127 encompasses the aperture 116. Toward its juncture with the base portion 120, the raised portion 122 includes a flared section 124 which extends around at least a portion of the raised portion 122. The flared section 124 provides a concavely curved transition between the base portion 120 and the raised portion 122. As best seen in Figures 20A and 21A, the circular base portion 120 projects radially outward beyond the raised portion 122 in every direction normal to axis 103. This shape allows the base portion to provide a maximum footprint area for attachment to the facet joint and compression across the joint, while the raised portion is reduced in size from the base portion, providing a minimally obtrusive device profile.

At least one connection feature which may be a recess is present on the cap. Cap 102 includes two connection features 126, 128 which are recessed into the raised portion 120, and provide a location for an instrument to grasp the cap 102. Connection features 126, 128 may cooperate with retaining members 564 of instrument 560 allowing instrument 560 to guide placement of cap 100 in an implantation procedure. The connection features are formed as slots in communication with the aperture. The flared section 124 may also provide a recessed surface for instrument connection. In other examples, the cap 102 may include one or more slots 24 for connection to a guiding or implantation instrument.

The attachment surface 114 includes a plurality of teeth 130 projecting inferiorly from the cap. The teeth 130 may vary in number and in distribution. Each tooth 130 may include at least one bevel 132. The bevels 132 may be oriented, or face interiorly toward, the center of the attachment surface 114. The positioning of the beveled teeth may provide compression across the joint as the cap 102 is affixed to the joint, as the beveled tooth surfaces on teeth on one side of the joint may oppose, or face, beveled tooth surfaces on teeth on the opposite side of the joint. Methods of implantation described above for cap 10 and fastener 50 also apply to devices 100, 200, 300, and 400.

Referring to Figures 19A and 19B, cap 102 is shown with screw 104. Screw 104 includes a head portion 140, a shaft portion 142, and is cannulated with a bore 144 extending the length of the screw. The head 140 includes a connection feature 146 which may be a hex connection or other type of connection which permits a driver to turn the screw 104. Screw 104 may be polyaxially adjustable relative to cap 102, with head 140 rotatable polyaxially within aperture wall 117. The second flared end 119 may allow space for the polyaxial tilting of the screw relative to the longitudinal axis of the aperture 116 and cap axis 103. Shaft portion 142 is at least partially threaded, with threads 148 occupying a portion of the shaft. It is appreciated that in other embodiments of device 100 and the other facet fixation devices herein, the screw 104 may be replaced with another type of fastener. For example, a rivet, brad, nail, bolt, post, peg, staple, anchor, line, flexible member, cable, wire or another type of fastener known in the art may be used to provide fixation of the cap across a facet joint. Screw 104 or other fastener may lock to the cap to prevent backout of the screw 104 or backout of the cap 102.

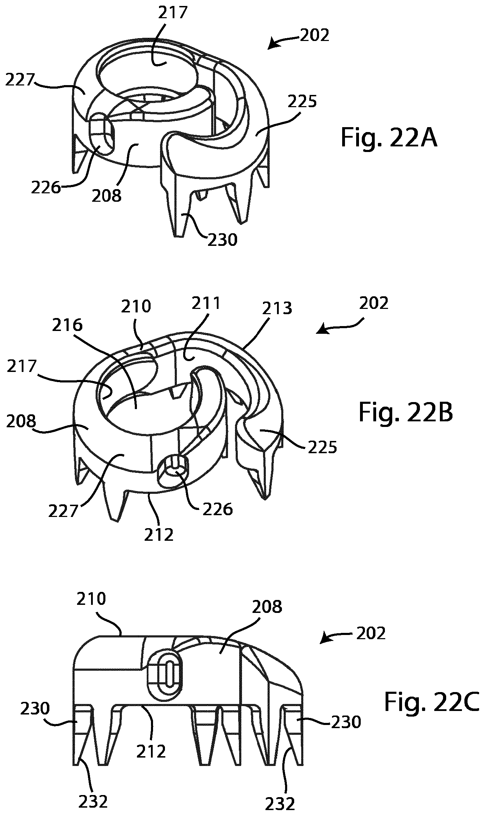

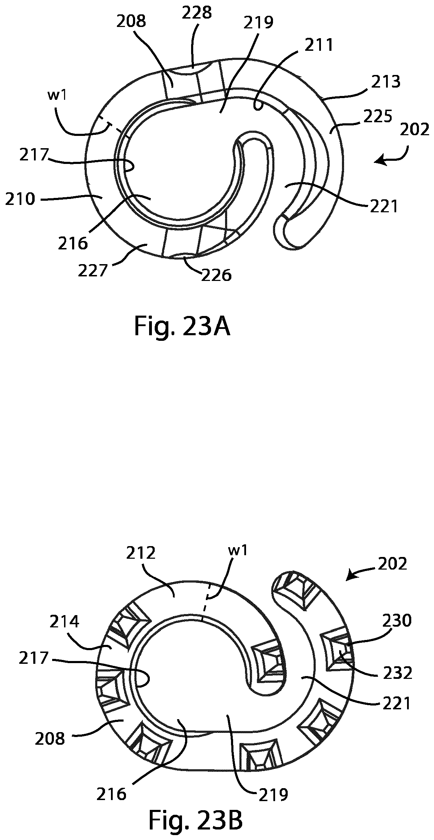

Referring to Figures 22A, 22B, 22C, 23A and 23B, an example of a facet fixation cap having a spiral shape is shown. Cap 202 is formed as a continuous snake-like shape, having a first end or lobe 225 which winds around and partially overlaps a second end or lobe 227. The spiral may be asymmetrically shaped, with an overall length of the cap greater than an overall width. Cap 202 includes a cap body 208 having a first side 210 opposite a second side 212; the cap also has an interior wall 211 opposite an exterior wall 213. The interior 211 and exterior 213 walls are generally perpendicular to the second side 212. As seen best in Figures 23A and 23B, the thickness, or width w1, of the cap body 208 between the interior wall 211 and the exterior wall 213 is constant throughout the cap body; in other examples the width of the cap body may vary at different locations. Exterior wall 213 may be smoothly rounded to provide a low unobtrusive profile when the cap 202 is implanted.

Cap 202 further includes an attachment surface 214 having a plurality of teeth 230 with bevels 232. As with cap 102, the positioning of the teeth and bevels promotes compression across the facet joint when the cap 202 is implanted with some teeth on one side of the joint, and the remaining teeth on the other side of the joint. An aperture 216 having a surrounding aperture wall 217 is formed in the second lobe 227; in the example the aperture 216 is not entirely closed off but a first gap 219 is formed in the aperture wall 217. First and second connection features 226, 228 are formed as recesses in cap body 208, on the exterior wall 213. It is appreciated that one or more connection features may be formed on any part of the cap body 208 in any location. The connection features are formed as slots in communication with the aperture The cap 202 may include one or more slots 24 for connection to a guiding or implantation instrument. A second gap is formed between the first lobe 225 and the second lobe 227. The first and second gaps 219, 221 may be referred to as fenestrations, and provide space for insertion of bone graft material.

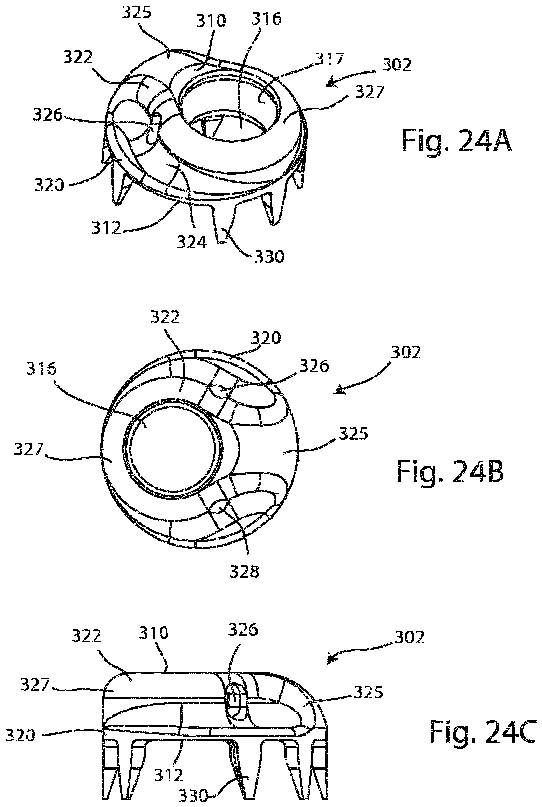

Referring to Figures 24A, 24B, and 24C, another embodiment of a facet fixation device is shown. Like device 100, a facet fixation cap 302 has a circular footprint. Cap 302 shares many of the characteristics of cap 102, which are numbered similarly, including a first side 310, second side 312, aperture 316, aperture wall 317, attachment surface 314, base portion 320, raised portion 322, flared section 324, beveled teeth 330, and connection features 326, 328, among others. Similar features shown and described for device 102 can be assumed to also apply to cap 302. While the base portion 320 is circular, the raised portion 322 is not radially symmetrical and has a non-circular, eccentric perimeter. The shape of the raised portion 322 differs from the shape of the raised portion 122 of cap 102 in that the relative sizes of the lobes of the raised portion are reversed. A second end, or lobe 327, which encompasses aperture 316, is wider than a first end, or lobe 325. Raised portion 322 has a non-circular, eccentric perimeter, while base portion 320 has a circular perimeter. The connection features may be formed as slots in communication with the aperture. Cap 302 may include one or more slots 24 for connection to a guiding or implantation instrument. In alternate embodiments, cap 302 may include a second aperture 41 which is a fenestration to provide a graft pocket for material such as demineralized bone material, graft material, bone chips or bone growth promoters.

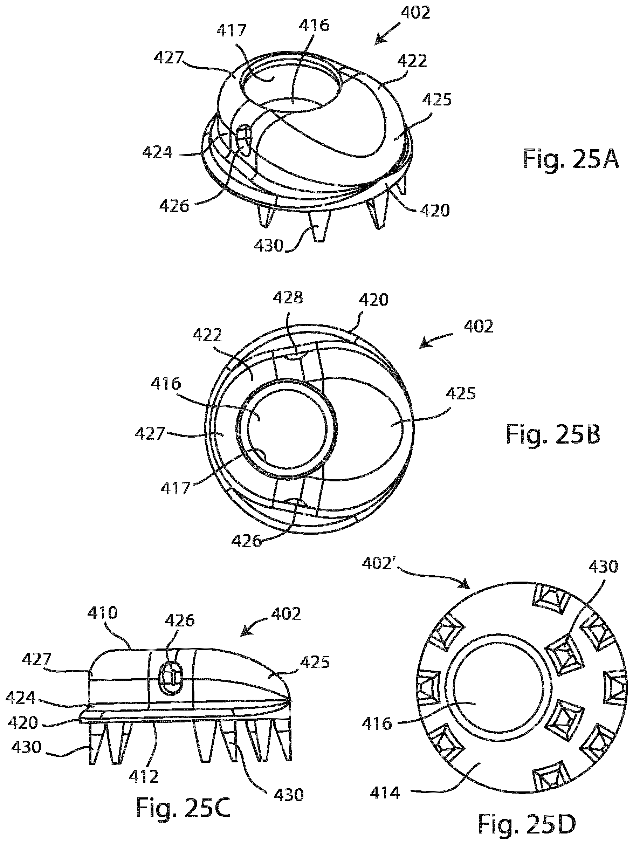

Referring to Figures 25A, 25B, and 25C yet another embodiment of a facet fixation device is shown. Like caps 102 and 302, a facet fixation cap 402 has a circular footprint. Cap 402 shares many of the characteristics of cap 102, which are numbered similarly, including a first side 410, second side 412, aperture 416, aperture wall 417, attachment surface 414, base portion 420, raised portion 422, flared section 424, beveled teeth 430, first and second ends 425, 427, and connection features 426, 428, among others. Similar features shown and described for device 102 can be assumed to also apply to cap 402. The shape of the raised portion 422 differs from the shape of the raised portion 122 of cap 102 in that the raised portion 422 is wider overall, for example between the connection features, compared to raised portion 122 of cap 102. Raised portion 422 of cap 402 overlays more of the base portion 420 than does raised portion 122 of base portion 120 of cap 102; portion 422 may be described as wider or beefier than raised portion 122. Raised portion 422 has a non-circular eccentric perimeter, while base portion 420 has a circular perimeter. A flared section 424 of cap 402 may be less dramatically curved than the flared section 124 of cap 102. In alternate embodiments, the connection features may be formed as slots in communication with the aperture, or as protruding bosses or tabs. In other examples, cap 402 may include one or more slots 24 for connection to a guiding or implantation instrument. In alternate embodiments, cap 402 may include a second aperture 41 which is a fenestration to provide a graft pocket for material such as demineralized bone material, graft material, bone chips or bone growth promoters.

Referring to Figure 25D, cap 402' is an alternative embodiment of cap 402. Cap 402' shares many of the same features as cap 402; however the number and pattern of teeth 430 projecting from attachment surface 414 differs. Cap 402' has two teeth 430 on the second end 425 between an outer row of teeth and the aperture 416, creating an outer perimeter of teeth and an inner row of teeth. In comparison, cap 402 has only one tooth 430 between an outer row and the aperture. It is appreciated that in any of the embodiments disclosed herein, the number and pattern of teeth may vary; teeth may be distributed in rows, groups, or singly; randomly or regularly; symmetrically or asymmetrically.

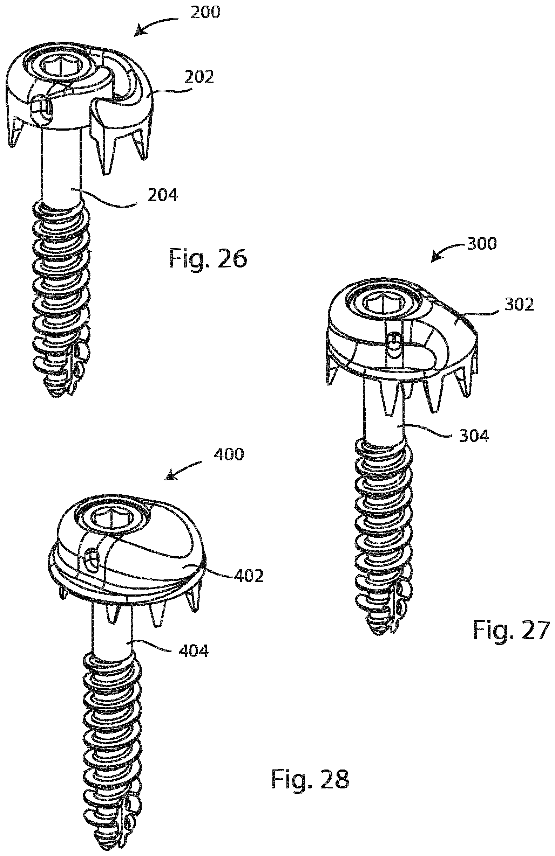

Referring to Figure 26, a facet fixation device 200 includes cap 202 and screw 204. Screw 204 may be the same as screw 104 and as such will not be further described. Screw 204 may be polyaxially adjustable relative to cap 202. The discontinuous spiral shape of cap 202 may allow some conforming of cap 202 to the targeted joint members when cap 202 is implanted. For example, the attachment surface 214 on lobe 225 may not remain coplanar with attachment surface 214 on lobe 227 as device 200 is implanted; some relative movement may occur between lobe 225 and lobe 227.

Referring to Figure 27, a facet fixation device 300 includes cap 302 and screw 304. Screw 304 may be the same as screw 104 and as such will not be further described. Screw 304 may be polyaxially adjustable relative to cap 302.

Referring to Figure 28, a facet fixation device 400 includes cap 402 and screw 404. Screw 404 may be the same as screw 104 and as such will not be further described. Screw 404 may be polyaxially adjustable relative to cap 402.

It should be understood that the present components, systems, kits and apparatuses are not intended to be limited to the particular forms disclosed. Rather, they are intended to include all modifications, equivalents, and alternatives falling within the scope of the claims. They are further intended to include embodiments which may be formed by combining features from the disclosed embodiments, and variants thereof.

The claims are not to be interpreted as including means-plus- or step-plus-function limitations, unless such a limitation is explicitly recited in a given claim using the phrase(s) "means for" or "step for," respectively.

The term "coupled" is defined as connected, although not necessarily directly, and not necessarily mechanically.

The use of the word "a" or "an" when used in conjunction with the term "comprising" in the claims and/or the specification may mean "one," but it is also consistent with the meaning of "one or more" or "at least one." The term "about" means, in general, the stated value plus or minus 5%. The use of the term "or" in the claims is used to mean "and/or" unless explicitly indicated to refer to alternatives only or the alternative are mutually exclusive, although the disclosure supports a definition that refers to only alternatives and "and/or."

The terms "comprise" (and any form of comprise, such as "comprises" and "comprising"), "have" (and any form of have, such as "has" and "having"), "include" (and any form of include, such as "includes" and "including") and "contain" (and any form of contain, such as "contains" and "containing") are open-ended linking verbs. As a result, a device that "comprises," "has," "includes" or "contains" one or more steps or elements, possesses those one or more steps or elements, but is not limited to possessing only those one or more elements.

## Claims

1. A device (100, 300, 400) for facet joint fixation, the device comprising:
a fastener; and
a cap (102), the cap (102) including: a base portion (120) having a circular perimeter; a raised portion (122) having a non-circular perimeter protruding from the base portion (120), the raised portion (122) including a first lobe (125) and a second lobe (127), the first lobe (125) wider than the second lobe (127), the widths of the first lobe (125) and second lobe (127) measured in a plane perpendicular to a cap axis (103); and
an aperture (116) extending through the base portion (120) and the raised portion (122), the aperture (116) shaped to receive the fastener to allow the fastener to fasten the cap (102) to a facet joint to stabilize the facet joint,
wherein the cap axis (103) is centrally located relative to the aperture (116),
wherein the base portion (120) includes a plurality of teeth (130) projecting from an attachment surface (114) opposite the raised portion (122);
**characterized by** the raised portion of the cap (102) including one or more connection features shaped to receive a guiding instrument;
wherein the one or more connection features are recesses (126, 128) or slots (18, 24) in communication with the aperture (116).

2. The device of claim 1, wherein the aperture (116) is offset from the center of the base portion (120).

3. The device of claim 1 or claim 2, wherein the aperture (116) extends through the first lobe (125).

4. The device of any preceding claim, wherein the fastener is a screw (104, 304, 404), wherein the screw (104, 304, 404) is polyaxially adjustable relative to the cap (102).

5. The device of any preceding claim, wherein each of the plurality of teeth has at least one beveled surface (132).

6. The device of claim 5, wherein the plurality of teeth (130) are arranged on the attachment surface (114) so that the beveled surfaces (132) are oriented inwardly, and wherein at least two opposing beveled surfaces (132) face one another to promote compression across the facet joint when the cap (102) is fastened to the facet joint,
optionally wherein the at least two opposing beveled surfaces (132) are on opposing sides of the aperture (116).

7. The device of any preceding claim, wherein the aperture (116) is circumscribed by an aperture wall (117), wherein the aperture wall (117) is spherically concave.

8. The device of claim 7, wherein the aperture wall (117) has a first end intersecting the raised portion (122) and a second end intersecting the attachment surface (114), wherein the aperture wall (117) widens at at least one of the first end and the second end.

9. The device of any preceding claim, wherein the connection feature comprises a pair of recesses (126, 128), and wherein the recesses (126, 128) are on opposite sides of the aperture (116).

10. The device of any preceding claim, wherein the perimeter of the raised portion (122) is eccentric.

11. The device of any preceding claim, wherein the raised portion (122) is ovoid shaped.

12. The device of any preceding claim, wherein the cap (102) further includes a flared section (124) at the juncture of the base portion (120) and the raised portion (122), the flared section (124) providing a curved transition between the base portion (120) and the raised portion (122).

13. The device of any preceding claim, wherein the cap (102) includes a cap axis (103) centrally located relative to the aperture (116), and the base portion (120) projects radially outward beyond the raised portion (122) in every direction normal to the axis (103).

14. The device of any preceding claim, wherein the cap (102) further includes a fenestration (41), the fenestration (41) providing a pocket for graft material.

15. The device of claim 14, wherein the base portion (120) includes an attachment surface (114) opposite the raised portion (122) and the attachment surface (114) is planar, and wherein the raised portion (122) is smoothly rounded to provide a low and minimally obtrusive device profile.

## Patentansprüche

1. Vorrichtung (100, 300, 400) zur Facettengelenkfixierung, wobei die Vorrichtung umfasst:
ein Befestigungselement; und
eine Kappe (102), wobei die Kappe (102) umfasst: einen Basisabschnitt (120) mit einem kreisförmigen Umfang; einen erhabenen Abschnitt (122) mit einem nicht kreisförmigen Umfang, der von dem Basisabschnitt (120) vorsteht, wobei der erhabene Abschnitt (122) eine erste Nase (125) und eine zweite Nase (127) umfasst, wobei die erste Nase (125) breiter ist als die zweite Nase (127), wobei die Breiten der ersten Nase (125) und der zweiten Nase (127) in einer Ebene senkrecht zu einer Kappenachse (103) gemessen sind; und
eine Öffnung (116), die sich durch den Basisabschnitt (120) und den erhabenen Abschnitt (122) erstreckt, wobei die Öffnung (116) geformt ist, um das Befestigungselement aufzunehmen, um zu ermöglichen, dass das Befestigungselement die Kappe (102) an einem Facettengelenk befestigt, um das Facettengelenk zu stabilisieren,
wobei die Kappenachse (103) mittig relativ zur Öffnung (116) angeordnet ist,
wobei der Basisabschnitt (120) eine Vielzahl von Zähnen (130) umfasst, die von einer Befestigungsfläche (114) gegenüber dem erhabenen Abschnitt (122) vorstehen;
**dadurch gekennzeichnet, dass** der erhabene Abschnitt der Kappe (102) ein oder mehrere Verbindungsmerkmale umfasst, die geformt sind, um ein Führungsinstrument aufzunehmen;
wobei das eine oder die mehreren Verbindungsmerkmale Vertiefungen (126, 128) oder Schlitze (18, 24) in Verbindung mit der Öffnung (116) sind.

2. Vorrichtung nach Anspruch 1, wobei die Öffnung (116) von der Mitte des Basisabschnitts (120) versetzt ist.

3. Vorrichtung nach Anspruch 1 oder Anspruch 2, wobei sich die Öffnung (116) durch die erste Nase (125) erstreckt.

4. Vorrichtung nach einem der vorhergehenden Ansprüche, wobei das Befestigungselement eine Schraube (104, 304, 404) ist, wobei die Schraube (104, 304, 404) relativ zur Kappe (102) polyaxial einstellbar ist.

5. Vorrichtung nach einem der vorhergehenden Ansprüche, wobei jeder der Vielzahl von Zähnen mindestens eine abgeschrägte Fläche (132) aufweist.

6. Vorrichtung nach Anspruch 5, wobei die Vielzahl von Zähnen (130) auf der Befestigungsfläche (114) so angeordnet sind, dass die abgeschrägten Flächen (132) nach innen gerichtet sind, und
wobei mindestens zwei gegenüberliegende abgeschrägte Flächen (132) einander zugewandt sind, um die Kompression über das Facettengelenk zu fördern, wenn die Kappe (102) an dem Facettengelenk befestigt ist,
optional wobei sich die mindestens zwei gegenüberliegenden abgeschrägten Flächen (132) auf gegenüberliegenden Seiten der Öffnung (116) befinden.

7. Vorrichtung nach einem der vorhergehenden Ansprüche, wobei die Öffnung (116) von einer Öffnungswand (117) umgeben ist, wobei die Öffnungswand (117) kugelförmig konkav ist.

8. Vorrichtung nach Anspruch 7, wobei die Öffnungswand (117) ein erstes Ende, das den erhabenen Abschnitt (122) schneidet, und ein zweites Ende, das die Befestigungsfläche (114) schneidet, aufweist, wobei die Öffnungswand (117) an mindestens einem von dem ersten Ende und dem zweiten Ende breiter wird.

9. Vorrichtung nach einem der vorhergehenden Ansprüche, wobei das Verbindungsmerkmal ein Paar Aussparungen (126, 128) umfasst und wobei sich die Aussparungen (126, 128) auf gegenüberliegenden Seiten der Öffnung (116) befinden.

10. Vorrichtung nach einem der vorhergehenden Ansprüche, wobei der Umfang des erhabenen Abschnitts (122) exzentrisch ist.

11. Vorrichtung nach einem der vorhergehenden Ansprüche, wobei der erhabene Abschnitt (122) eiförmig geformt ist.

12. Vorrichtung nach einem der vorhergehenden Ansprüche, wobei die Kappe (102) ferner einen aufgeweiteten Abschnitt (124) an der Verbindungsstelle des Basisabschnitts (120) und des erhabenen Abschnitts (122) umfasst, wobei der aufgeweitete Abschnitt (124) einen gekrümmten Übergang zwischen dem Basisabschnitt (120) und dem erhabenen Abschnitt (122) bereitstellt.

13. Vorrichtung nach einem der vorhergehenden Ansprüche, wobei die Kappe (102) eine Kappenachse (103) umfasst, die mittig relativ zu der Öffnung (116) angeordnet ist, und der Basisabschnitt (120) radial nach außen über den erhabenen Abschnitt (122) hinaus in jede Richtung senkrecht zur Achse (103) vorsteht.

14. Vorrichtung nach einem der vorhergehenden Ansprüche, wobei die Kappe (102) ferner eine Fensterung (41) umfasst, wobei die Fensterung (41) eine Tasche für Transplantatmaterial bereitstellt.

15. Vorrichtung nach Anspruch 14, wobei der Basisabschnitt (120) eine Befestigungsfläche (114) gegenüber dem erhabenen Abschnitt (122) umfasst und die Befestigungsfläche (114) eben ist und wobei der erhabene Abschnitt (122) glatt abgerundet ist, um ein niedriges und minimal vorstehendes Vorrichtungsprofil bereitzustellen.

## Revendications

1. Dispositif (100, 300, 400) destiné à la fixation d'articulation facettaire, le dispositif comprenant :
un élément de fixation ; et
un capuchon (102), le capuchon (102) comprenant : une partie de base (120) possédant un périmètre circulaire ; une partie surélevée (122) possédant un périmètre non circulaire faisant saillie à partir de la partie de base (120), la partie surélevée (122) comprenant un premier lobe (125) et un second lobe (127), le premier lobe (125) étant plus large que le second lobe (127), les largeurs du premier lobe (125) et du second lobe (127) étant mesurées dans un plan perpendiculaire à un axe de capuchon (103) ; et
une ouverture (116) s'étendant à travers la partie de base (120) et la partie surélevée (122), l'ouverture (116) étant façonnée pour recevoir l'élément de fixation pour permettre à l'élément de fixation de fixer le capuchon (102) à une articulation facettaire pour stabiliser l'articulation facettaire,
ledit axe de capuchon (103) étant situé de manière centrale par rapport à l'ouverture (116),
ladite partie de base (120) comprenant une pluralité de dents (130) faisant saillie à partir d'une surface d'attache (114) opposée à la partie surélevée (122) ;
**caractérisé par** la partie surélevée du capuchon (102) comprenant un ou plusieurs éléments de raccordement façonnés pour recevoir un instrument de guidage ;
ledit ou lesdits éléments de raccordement étant des évidements (126, 128) ou des fentes (18, 24) en communication avec l'ouverture (116).

2. Dispositif selon la revendication 1, ladite ouverture (116) étant décalée du centre de la partie de base (120).

3. Dispositif selon la revendication 1 ou la revendication 2, ladite ouverture (116) s'étendant à travers le premier lobe (125).

4. Dispositif selon une quelconque revendication précédente, ledit élément de fixation étant une vis (104, 304, 404), ladite vis (104, 304, 404) étant réglable de manière polyaxiale par rapport au capuchon (102).

5. Dispositif selon une quelconque revendication précédente, chacune de la pluralité de dents possédant au moins une surface biseautée (132).

6. Dispositif selon la revendication 5, ladite pluralité de dents (130) étant agencées sur la surface d'attache (114) afin que les surfaces biseautées (132) soient orientées vers l'intérieur, et
au moins deux surfaces biseautées opposées (132) se font face pour favoriser la compression à travers l'articulation facettaire lorsque le capuchon (102) est fixé à l'articulation facettaire,
éventuellement lesdites au moins deux surfaces biseautées opposées (132) étant sur des côtés opposés de l'ouverture (116).

7. Dispositif selon une quelconque revendication précédente, ladite ouverture (116) étant circonscrite par une paroi d'ouverture (117), ladite paroi d'ouverture (117) étant concave de manière sphérique.

8. Dispositif selon la revendication 7, ladite paroi d'ouverture (117) possédant une première extrémité coupant la partie surélevée (122) et une seconde extrémité coupant la surface d'attache (114), ladite paroi d'ouverture (117) s'élargissant au niveau d'au moins l'une de la première extrémité et de la seconde extrémité.

9. Dispositif selon une quelconque revendication précédente, ledit élément de raccordement comprenant une paire d'évidements (126, 128), et lesdits évidements (126, 128) étant sur les côtés opposés de l'ouverture (116).

10. Dispositif selon une quelconque revendication précédente, ledit périmètre de la partie surélevée (122) étant excentrique.

11. Dispositif selon une quelconque revendication précédente, ladite partie surélevée (122) étant de forme ovoïde.

12. Dispositif selon une quelconque revendication précédente, ledit capuchon (102) comprenant en outre une section évasée (124) au niveau de la jonction de la partie de base (120) et de la partie surélevée (122), la section évasée (124) fournissant une transition incurvée entre la partie de base (120) et la partie surélevée (122).

13. Dispositif selon une quelconque revendication précédente, ledit capuchon (102) comprenant un axe de capuchon (103) situé de manière centrale par rapport à l'ouverture (116), et ladite partie de base (120) faisant saillie radialement vers l'extérieur au-delà de la partie surélevée (122) dans toutes les directions normales à l'axe (103).

14. Dispositif selon une quelconque revendication précédente, ledit capuchon (102) comprenant en outre un fenêtre (41), le fenêtre (41) fournissant une poche pour le matériau de greffe.

15. Dispositif selon la revendication 14, ladite partie de base (120) comprenant une surface d'attache (114) opposée à la partie surélevée (122) et ladite surface d'attache (114) étant plane, et ladite partie surélevée (122) étant légèrement arrondie pour fournir un profil de dispositif bas et à peine intrusif.
